# EUROPEAN PATENT SPECIFICATION

(11) **EP 1 324 727 B1**
(45) Date of publication and mention of the grant of the patent: **24.11.2004**
(21) Application number: 01972353.5
(22) Date of filing: 09.10.2001
(51) Int. Cl.: A61F 5/00, A61F 5/56

(54) **OBESITY TREATMENT AID**
HILFSVORRICHTUNG ZUR BEHANDLUNG DER FETTLEIBIGKEIT
DISPOSITIF D'AIDE AU TRAITEMENT DE L'OBESITE

(30) Priority: 13.10.2000 GB 0025116; 28.07.2001 GB 0118493
(43) Date of publication of application: 09.07.2003
(73) Proprietor: Hall, Richard M., Leeds LS17 8ER (GB)
(72) Inventor: Hall, Richard M., Leeds LS17 8ER (GB)
(74) Representative: Denmark, James
(86) International application number: PCT/GB2001/004475
(87) International publication number: WO 2002/030341

(56) References cited:
- EP-A- 0 254 918
- CH-A- 675 677
- US-A- 4 595 361

## Description

This invention relates to a device for treating obesity in humans, and more specifically to a device which can be applied within the mouth of a human to restrict the intake of food and thus reduce obesity.

### INTRODUCTION AND BACKGROUND

### Obesity and its Complications

Obesity is a major health and social problem worldwide for which no single satisfactory treatment exists. In Westernised society there is an epidemic prevalence of the disease with 20% of the UK population grossly overweight and recent calculations showing that more than a third of US adults (97 million) are now obese as are 25-30% of US children. People with normal weight have a Body Mass Index (BMI) of 20-25. (BMI = weight in kilograms divided by the square of the height in metres). Overweight people have a BMI of 25-30 and the obese a BMI over 30.

The most common reasons seen in obesity clinics for patients wishing to lose weight among both men and women are appearance, social embarrassment, physical symptoms including general discomfort, shortness of breath and pain in the weight bearing joints. Severely obese patients with psychiatric disorders are difficult to treat by diet alone. Patients who eat for consolation find it significantly more difficult to achieve and maintain weight loss than others. There is evidence of a relationship between obesity and depression. Obesity is associated with a number of medical conditions including diabetes, high blood pressure, risk of stroke and coronary and heart disease, osteoarthritis and chest disorders. Obese people therefore have a risk of reduced life expectancy.

The problem of treatment of moderate obesity is to find an effective approach which is efficient in maintaining reduced weight and the objective of surgical and non-surgical intervention is to encourage or force the patient to diet. Restricted dietary intake can produce weight loss in the short to medium term. However, weight is generally regained when the treatment is stopped. Weight loss is not rapid, the optimum being about 1kg per week, although losses of 10-15kg have been achieved after 6 months especially associated with nutritional education and behaviour modifications. After weight reduction patients may attempt to prevent weight regain by applying a tight nylon waist cord which provides a psychological and physical barrier. The obese public are prepared to spend a great deal of money on weight loss programmes (£2000-£3000). Treatment also involves the nutrition/diet industry. A variety of drugs have been tried as adjuncts to diet therapy but have only proven helpful in the short-term and continued drug therapy not only has side effects but is also expensive.

Jaw wiring (maxillomandibular fixation or MMF) is a more aggressive treatment to restrict solid food intake which is effective but does not always result in long term weight loss as many patients rapidly regain weight having reverted to previous eating habits once the jaw fixation is removed. There are reported success treatments for some patients after MMF followed by use of a nylon waist cord. Jaw wiring can produce a panic fear reaction (tension and anxiety regarding the procedure and risk of choking) which results in the patient defaulting by unwiring the jaws themselves. In many cases, jaw wiring can give rise to dental health complications such as episodes of periodontal pain and mandibular limitations of movement after removal of the fixation. Where obese patients are edentuless in one or both jaws, dentures are secured under general anaesthetic to the edentulous jaws by various wiring methods and the jaws immobilised by inter-dental wires where teeth are present and inter-maxillary wires where they are not. The mean fixation duration of jaw wiring is average 7 months and MMF has been used up to 18 months.

To date, the only completely successful treatment with long-lasting effects for reduced food intake is surgical intervention in the gastro-intestinal tract eg stomach stapling or stomach reduction, surgical bypass, banding procedures, laproscopic gastric banding. This approach for the severely obese patient increases surgical and mortality risk as surgical operations of this type require general anaesthetic and many of these patients already have medical problems relating to their condition. There are also direct surgical problems related to the excess body fat as well as serious complications to gastric bypass surgery which involve various types of malabsorption of food. There is currently a 2 year waiting list for patients to be seen on the obesity clinic at the Leeds General Infirmary in the UK with 200 patients waiting for surgical procedures.

Occlusal splints are used by dental surgeons to treat a wide range of dental problems and are custom made using heat cured acrylic resin or soft vacuum formed materials. They are used in either upper (maxillary) or lower (mandibular) jaws, cover all surfaces of the teeth, and can be fitted and removed by the patient or their dentist as desired. Applications for such occlusal splints include:
- Control of tooth wear as night guards where there are grinding habits with associated muscle discomfort;
- protection of restorations and avoidance of loosening cemented crowns;
- Management of jaw joint problems which are often associated with stress and psychological causes;
- Mobile teeth stabilisers following orthodontic or periodontal treatment;
- Diagnosis of bite problems such as bite interferences, tooth migration, soft tissue trauma, reduced vertical dimensions of the jaws through tooth wear;
- To assist grossly overweight patients reduce food and thus energy intake by making chewing difficult.

The last of these applications has been tried with only limited success.

In terms of prior art of which the applicant is currently aware, US4727867 describes a dental appliance consisting of a metal frame having a pair of wings adapted to be disposed between the teeth and the tongue of a wearer and which inhibit the movement of the jaws in a lateral direction so that chewing is made difficult by the fact that the jaws can only move towards and apart in a vertical direction. WO9742916 describes a similar device which is affixed to the outside of the teeth of upper and lower jaws to limit the extent of vertical separation thereof so that only a limited volume of food can be ingested.

US4471771 describes a dental fixing to which is pivotally mounted a mesh or grid which prevents the ingesting of solid foodstuffs but allows liquid and finely ground foods to pass therethrough thus limiting the consumption of food by a wearer. The pivot allows the mesh to be deflected upwardly in the event of regurgitation by the wearer. US5924422 describes a further oral device for slowing down the rate of food ingestion by lowering the effective volume of the normally vaulted portion in the roof of the mouth. The device consists of a moulded plastics piece which is secured to the maxillary jaw by wires around existing teeth therein so as not to prevent occlusion of the wearer.

EP0254918 teaches a device for curing or alleviating excessive snoring on the part of the wearer. Specifically the document discloses a set of caps or frames which are created from impressions of the upper and lower dentitions of their wearer having a pair of flexible webs which can be sprung outwardly to enable the frames to be disposed over the teeth of the wearer in snap-fitting manner. The flanges of the caps which join respective webs are provided with permanent magnets and pieces of ferromagnetic material respectively so that when the frames are in place on the wearers maxillary and mandibular dentitions, the permanent and ferromagnetic materials are superposed and magnetically attract one another.

It is clear from the description and drawings that the magnetic elements are disposed within the flanges of the frames which join the webs above the biting surfaces of the teeth so that the jaws of the wearer are maintained in a closed relationship to prevent snoring. There are no specific additional features provided to secure the frames to the teeth..

US4595361 describes a means of mounting magnetic elements to the outside or buccal sides of the teeth of a wearer. Specifically a kit is provided containing magnetic modules which are secured to the sides of the teeth of a wearer by means of metal bands on which are provided eyelets through which metal wires with non-circular cross-section are fed, one or more of the modules being mounted on the metal wires so as to be translatable thereon. This device is intended for orthodontic corrective treatment.

Finally, CH675677 describes a jaw position correction system comprising a pair of plates for affixing to the maxillary and mandibular jaws behind the teeth to which permanent magnets are secured in the suitable locations in front of the teeth and to either side of the front of the mouth of the wearer. The permanent magnets are ideally secured to the plates by movable fixings which allow the magnets to be moved around to provide the most suitable corrective force as the jaws approach each other. It is to be mentioned that the permanent magnets used in this invention continue to permit the conventional motion of the jaws and the lateral positioning of the magnets is important as it is the amount of offset distance between the magnets on the upper and lower plates which ultimately determines the amount of correction in jaw positions which will be achieved.

It is an object of the invention to provide a dental occluding system which effectively reduces the ability of a human to ingest food and which thus act to reduce the weight of said human over a period of time while nevertheless offering a facility for reducing, minimising or eliminating the efficacy of the device in the event of emergency.

It is a further object of the invention to provide a magnetic dental device for the fully dentate or partially edentuless patient which have minimum bulk and are not displaced by chewing or magnetic forces.

According to the present invention there is provided a dieting aid comprising at least one pair of frames which are capable of being secured over portions of the maxillary and mandibular dentitions respectively of a wearer, said frames being created using impressions of the maxillary and mandibular jaws so the resulting frames fit snugly over the plurality of teeth for which said frames are made, characterised in that inter-dentally extensible and retractable screws are provided in mountings on one side of both frames for releasably securing the frames over said plurality of teeth together with magnetic means also mounted to one side of each of said frames in a disposition such that the magnetic means of the maxillary frame at least partially superpose the magnetic means of the mandibular frame when said frames are secured over the teeth within the mouth of a wearer when the jaws are occluded.

It is to be understood hereinafter that the use of the term "magnetic means" is to be read as including a combination of ferromagnetic material in which the magnetic dipoles are not aligned, that is unmagnetised material, and a ferromagnetic material in which the dipoles are aligned, that a magnetised ferromagnetic material. Hence, a simple magnet and keeper arrangement may be considered, as opposed to magnets being provided in each frame.

Preferably four frames are used, the frames being secured to the upper, lower, left and right rear dental quadrants. By this is meant the collection of molar and/or premolar teeth occupying the rearmost portion of the dentition to the in the upper and lower jaws.

Preferably, the polarities of the magnetic means are opposite on those sides of the magnetic means on the maxillary frame and the mandibular frame which are closest to one another when the frames are affixed within the mouth of a human. This ensures that the jaws remain magnetically occluded except in emergency situations where additional forces can be applied to separate the jaws, perhaps by using the fingers, or using a specially adapted separating device.

Alternately, the polarities of the magnetic means are alike on those sides of the magnetic means on the maxillary frame and the mandibular frame which are closest to one another when the splints are affixed within the mouth of a human. In this instance the jaws are repelled apart, which makes ingestion of food very tiring for a wearer because not only does the wearer have to apply muscular force to the jaws to chew food, but also that muscular force must overcome the magnetic repulsion, and therefore eating can be come very quickly tiring.

Most preferably the magnetic means employed in each frame include split pole magnets which provide closed field magnetism, said magnets in the maxillary and mandibular frames being angularly offset by substantially 180° so that the adjacent opposing polarity portions of each magnet in one of the maxillary or madibular frames approaches or is brought into contact with split pole magnet portions of opposite polarity in the alternate frame as the jaws are occluded.

Closed field magnetism has the advantages of effectively concentrating the magnetic force which holds the jaws together in proximate relationship when said jaws are occluded, and reducing the amount of magnetic flux surrounding the magnets to a minimum which is not used to attract the alternate split pole magnet in the alternate frame.

Additionally, the dependence of the magnetic force of attraction between the magnet pairs in the maxillary and mandibular frames on their relative separation increases dramatically so that said magnetic force is large when the magnets are very close and reduces dramatically with only small increments in separation. This allows the jaws to be separated more easily by imparting a slight impact to one or other of said jaws, as the impact will be effective at initially separating he magnets from one another and immediately after separation, the magnetic attraction is much reduced and the jaws can be widened by the wearer.

Most preferably the frames are cast according to the shape of the teeth of the wearer over which said frames are affixed.

According to a second aspect of the invention there is provided a dieting aid comprising at least one pair of frames which can be secured to the maxillary and mandibular jaws of a wearer by suitable means, each of said frames having secured thereto magnetic means in a disposition such that the magnetic means of the maxillary frame at least partially superpose the magnetic means of the mandibular frame when secured within the mouth of a wearer, characterised in that the frames are created using impressions of the maxillary and mandibular jaws so the resulting frames fit snugly over the plurality of teeth for which said frames are made and further characterised in that the frames are provided with inter-dentally extensible and retractable means capable of releasably securing the frames over said plurality of teeth.

It is preferable that said frames are cast from impressions taken of the upper and lower dentition which include the rearmost four molar/premolar teeth in the maxillary and mandibular jaws, known more commonly as a dental quadrant, and while the frames are preferably cast or galvano-formed (a recent process which can be conducted using a machine from Heraeus Kulzer GmbH), any process for forming the frames may be suitable, as long as that process uses as its starting point previously taken impressions of the jaws as abovementioned. Moulding processes may also be applicable. In the galvano-forming process, gold may be formed exceedingly thinly so the resulting frames will be lightweight and unlikely to cause any distress to the wearer.

Preferably the frames are cast in such a manner to provide interdentally extending nibs on the lingual side of said frames.

Preferably the polarities of the magnetic means on the maxillary frame and the mandibular frame are opposite on the surfaces thereof which are more proximate when the frames are in place within the mouth.

Preferably the magnetic means are split pole magnets as described above.

Preferably shoulder formations are provided partially or entirely around, or to one side of either the upper and lower magnetic means which are provided on superposed frames, the magnetic means provided on the alternate superposed frame abutting said shoulder formations when the jaws are in their occluded condition to prevent significant lateral movement thereof.

Most preferably the frames include buttresses which support the magnetic means which are ideally disposed on the buccal side of the frame as it is situated inside the mouth.

Most preferably the disposition and orientation of the magnetic means on each of the maxillary and mandibular frames is such that when in mating contact ensuring the occlusion of the jaws of the wearer, the so-called freeway space between the biting or occlusal surfaces of at least the anterior teeth is maintained. Most preferably when the jaws are in the occluded position a space of approximately 3mm on average is maintained between anterior tooth surfaces, this amount reducing towards the rear of the mouth. In this manner the capacity for speech is not significantly impaired and words spoken by the wearer can be easily understood.

This ensures that the wearer of the cast frames does not suffer any adverse jaw joint or muscle effects as a result of having their jaws occluded too closely together which can cause grinding of the teeth, or being unable to close their jaws to a comfortable rest position by virtue of the mating contact of the magnetic means and the cast frames.

It is yet further preferable that the frames substantially cover the external surfaces of the teeth of the dental quadrants over which said frames are secured.

Most preferably, the magnetic means are laser welded to the frames and are provided with a planar mating surface. Alternately, the magnetic means may be secured to the frame by being set in an acrylic or other curable compound disposed to one side of the frame. Yet further alternately, the magnetic means may be secured to the frame by means of swaging or soldering.

In a most preferred embodiment, the frames are provided with additional retention lugs on the buccal side of the frame in use by virtue of which a tooth coloured acrylic or other curable or setting compound can be secured to the frames, possibly to cover, surround or secure the magnetic means to the frame. Ideally such compound would be tooth or flesh coloured.

Most preferably, the frames consist of a tooth biting or occlusal surface cover portion, which can be solid or of a lattice-type construction, and a strap or side wall portion which depends from or extends upwardly of the cover portion depending on whether the frame is for affixing to a maxillary or mandibular jaw portion, said strap portion in use ideally being in contact with the necks of the teeth over which the frame is applied. Most preferably said strap portion is disposed beneath or above the cover portion (depending on which jaw the frame is affixed to) on the opposite side of said cover portion to that on which the interdental screws are disposed such that a lateral clamping effect is achieved therewith.

It is yet still further preferable that the magnetic means are replaceable, and further preferable that said magnetic means consist of magnets having a body which terminates in at least one substantially planar contact surface, said body being received in collet formations secured to the buccal side of the frames. Most preferably, the body of the magnets are provided with a screw thread which interengages with a corresponding thread on the inside surface of the collet formations on the frame.

In one embodiment, each of the frames for either or both of the upper and lower dentitions may be cast as a single piece and resiliently deformable so as to be capable of snap fitting to the lingual side of the teeth.

Most preferably the strap or side wall portion of the frame which extends away from the biting or occlusal surface cover portion of said frame allows for the undercut of the teeth in whose sides said strap is in contact with to take account of the barrel shape of the molar/premolar teeth above the gums.

Preferably the frames are cast in Titanium but any dentally approved metal, alloy, plastics or polymer composition may be used, including acrylic and ceramic type materials. Titanium has the advantages that it does not electrolytically interfere with metal already present in fillings on the biting or occlusal surface of teeth of the wearer, is very strong and lightweight, can be cast in thin sections, and can be welded. Additionally, Titanium may be anodised to give the metal an alternate surface appearance, in particular to change its colour. Gold may also be used in the casting process, or in a galvano-forming process as above mentioned.

It is also foreseen by the applicant that the device may be used as a snoring prevention device as its function in one aspect ensures that the jaws remain slightly open at all times when the frames are in place and a magnetic keeper is not used to significantly reduce the magnetic attraction between said frames. In the alternate aspect of the invention where the frames serve to maintain the jaws in spaced apart relationship, the device could be used to prevent sleep apnoea.

During sleep, the use of a keeper plate/stent to cover the magnets of either the upper or lower dental splints may be required so that the jaws are not forced apart from the natural resting position causing jaw joint/muscle problems. This has the potential disadvantage of allowing the patient to selectively close off the magnetic fields so that they can each more effectively and negate the action of the splints to control dietary intake.

Where an additional magnetic keeper is employed over the magnetised magnetic means, the wearer can be allowed to exercise the jaws for a predetermined period as the magnetic attraction between each set of magnetic means is either substantially reduced or eliminated.

A dental filler, cement or reline material may preferably be applied to the fitting surface of the frames which are then subsequently applied over the specific dental quadrants to increase the comfort of said frames, their retention on the dental quadrants and stability thereon, and prevent the frames from damaging tooth enamel while in place. The filler or reline material can also assist in the prevention of tooth decay.

In this regard, it may be possible to securely mount the frames to the teeth using a combination of only a single inter-dentally extending screw and temporary dental cement or other adhesive compound.

In a most preferred embodiment, vented insert means may be provided for fitting between the anterior teeth of maxillary and mandibular jaws. This serves to prevent any over-eruption of the anterior teeth as a result of the lack of contact of their biting surfaces over the period when the frames are being worn. The insert may take the form of a vented resilient bung, or alternatively the frames may be disposed over, behind or in front of the entire dentitions of maxillary and mandibular jaws but in a much reduced or hidden manner in the region of the anterior teeth, and in this region small spheres may be cast onto the frames so that when the jaws are occluded, the spheres provide a contact surface for the anterior teeth of either the upper or lower dentitions, depending on which frame the spheres are provided. The spheres thus provide a contact for the anterior teeth between their biting edges and the cingulum area thereof.

Frame design must allow easy daily patient oral hygiene maintenance with no compromise to dental health and must remain functionally active for the planned duration of treatment which can be 28 days to 3 months or more.

The rationale behind the novel idea of using a magnetic dental splint system to aid weight loss is to avoid all the complications of permanent jaw wiring whilst at the same time restricting food intake by making it either uncomfortably tiring for patients to eat or keep the jaws closed through a magnet connection which allows slight sliding movement. The frames should be able to be used as an economic standalone or adjunctive treatment for many more obese patients than gastric operative bypass procedures, or as a pre-operative measure to help weight loss where there is morbid obesity so as to reduce the mortality risk from surgery as well as allow improved post-operative healing by reduction of excess fat tissue.

Opposite polarity paired magnets or a magnet-keeper format may be used as mentioned above. Attraction forces from this coupling will keep the jaws closed but allow a small sliding movement so that the patient does not feel totally locked which should avoid panic attacks.

Repulsion forces from the like pole magnetic coupling make chewing difficult as the magnets would want to force the jaws apart as well as having the advantage of helping to automatically self-seat the upper and lower dental frames on the teeth. Another benefit of this magnet coupling is that it should allow normal speech and avoid emergency choking and panic situations. This is therefore the optimum approach but the small magnets will have to overcome minimum natural biting force of over 20 kgm.

Thus the invention overcomes the negative aspects of previously practised jaw-wiring techniques, particularly as regards the possible fatality of a person inhaling regurgitated matter where it is impossible for them to open their jaws in such event while being a non-invasive technique and without impairing dental quality.

A specific embodiment of the invention will now be described by way of example with reference to the accompanying diagrams wherein
Figure 1 shows a schematic sectional view through the jaws of a wearer of cast frames according to the invention;

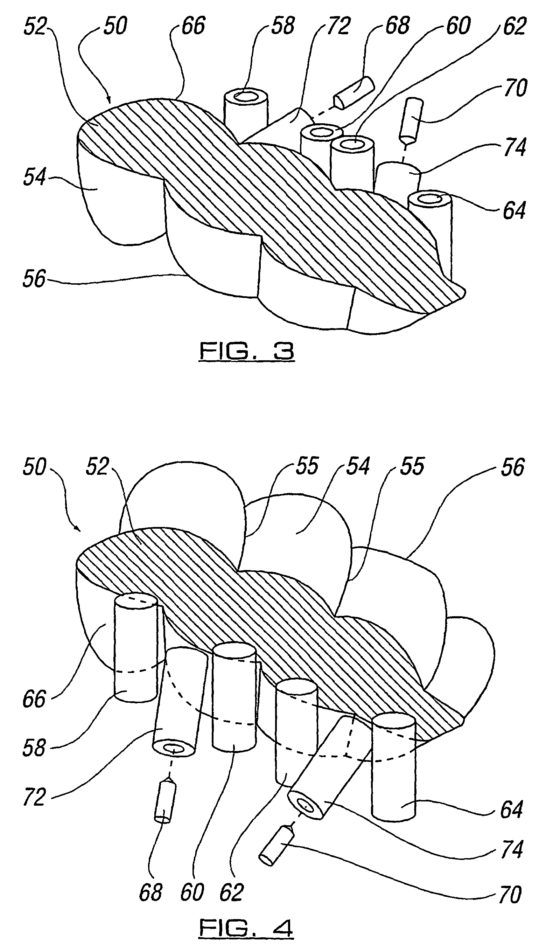

Figure 2 shows a schematic cheek or buccal side elevation of the maxillary jaw of a wearer having a cast frame secured thereto;
Figure 3 shows a perspective view of an actual cast frame specifically cast according to an impression of the mandibular jaw of a wearer, and in particular the dental quadrant of that jaw;
Figure 4 shows a perspective view from below the cast frame of Figure 3, and
Figure 5 shows schematically how the frame may be rotationally fitted over the teeth in a dental quadrant of one jaw of a wearer.

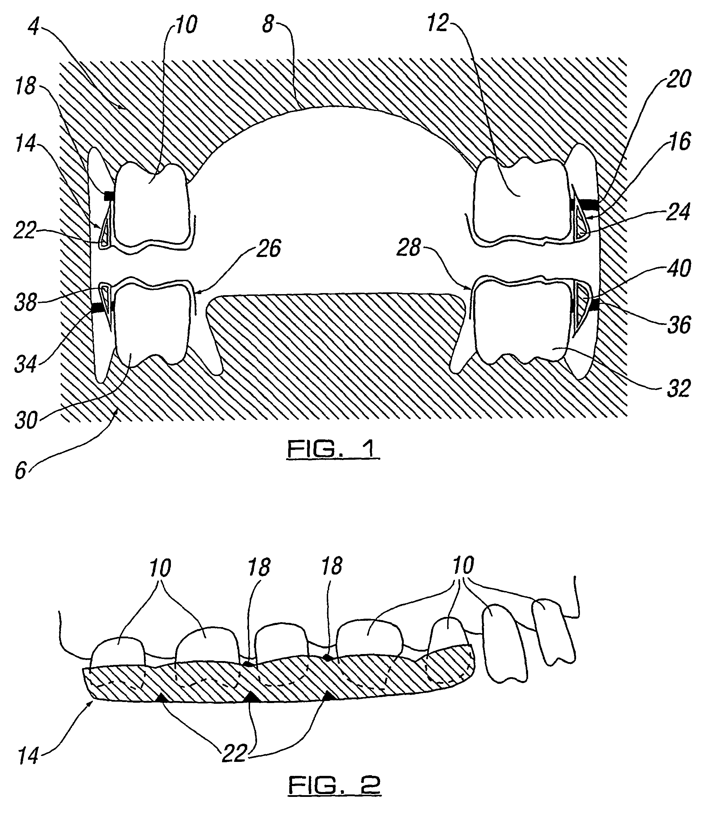

Referring firstly to Figure 1 there is shown a maxillary dentition 4 and a mandibular dentition 6, said maxillary dentition having a palate 8 and teeth 10, 12 to which cast frames 14, 16, are secure by means of interdental screws 18, 20. The frames 14, 16 are ideally separate or may form part of a single maxillary frame, but in any event each of said frame 14, 16 is provided with a magnet 22, 24 whose orientation is selected according to the desired direction of magnetic attractive or repulsive force.

The mandibular dentition 6 is additionally provided with frames 26, 28 also being secured to teeth 30, 32 by means of interdental screws 34, 36, and each of said frames 26, 28 is also provided with magnets 38, 40 whose polarities are either alike or opposite to the direction of the polarities of the magnets 22, 24 provided in the maxillary frames.

In a most preferred arrangement the magnets are disposed to the buccal side of the teeth 10, 12, 30, 32 so that the magnets of the maxillary frame attract or repel the magnets of the mandibular frame, and most preferably there is an attractive force between the maxillary and mandibular frames so that the jaws are occulded together by magnetic attraction forces.

If a wearer of the frames is desirous of consuming solid foods, the muscular force required to separate the magnets of the splints and thus the jaws is ideally less than the force of magnetic attraction, and the wearer cannot therefore separate the jaws without applying additional separating force to the jaws, for example by using his hands or a tool, such as a spoon, between the anterior teeth. This may be used in the event of a panic attack or where a possibility of choking arises. Alternately, where the magnets repel each other, the force required to occlude the jaws as they approach one another in a chewing action is significantly increased and thus the muscles causing actuation of the jaws wearer become quickly tired, with the result that the wearer quickly becomes uninterested in food consumption.

Minimum occlusal (biting surface) coverage of the teeth by the frames is essential so as not to interfere with the natural freeway space of the upper and lower teeth (2-3mm) which if constrained to be less or more than this amount could cause jaw joint and muscle problems. The frames are ideally fitted and removed by trained, accredited dental surgeons. On completion of treatment as well during treatment for dental health maintenance purposes the splints must be able to be removed without damage to the teeth. The use of inter-dental wedge-type screws 18 strategically placed is necessary to give enhanced mechanical retention.

Suitable metals for splint fabrication include cast titanium, chrome cobalt, and suitable non-metal materials include polymers, plastics and silicone, acrylics, but in any event the important requirement is that the material chosen must be biocompatible and approved for medical device use with similar thermal expansion to the teeth and able to withstand autoclave temperatures of 135°C for sterilisation. The materials used should ideally allow the frame to closely adapt to the tooth contour during fitting. The encapsulated magnets will be based on the established technology of the Technovent MAGNA-CAP® Attachment System which has full regulatory approval for medical/dental applications via long-standing clinical validation.

A keeper stent may be required during fitting of the splints for the closed jaw approach and for night use if the open jaw configuration is used so as to allow the muscles to be relaxed and the jaws not forced open.

Referring now to Figure 3, there is shown a cast frame 50 which is ideally suited for fitting to the right dental quadrant of a mandibular jaw, that is where the frame 28 is fitted in Figure 1. This frame is provided with a lattice 52 (which in most cases is more preferably be a solid construction) which in use covers the biting or occlusal surface of the teeth and from said lattice depends a lingual side wall portion 54 which terminates in a side wall lower edge 56. This side wall portion 54 bulges slightly in the region where it surrounds the teeth enclosed by the frame to define nibs 55 which extend interdentally and furthermore the lower edge 56 is inwardly disposed with respect to the zenith of the bulged surfaces of the side wall such that the undercut of the tooth profile is accommodated and the frame is prevented from slipping over the teeth encased thereby when in position in the mouth.

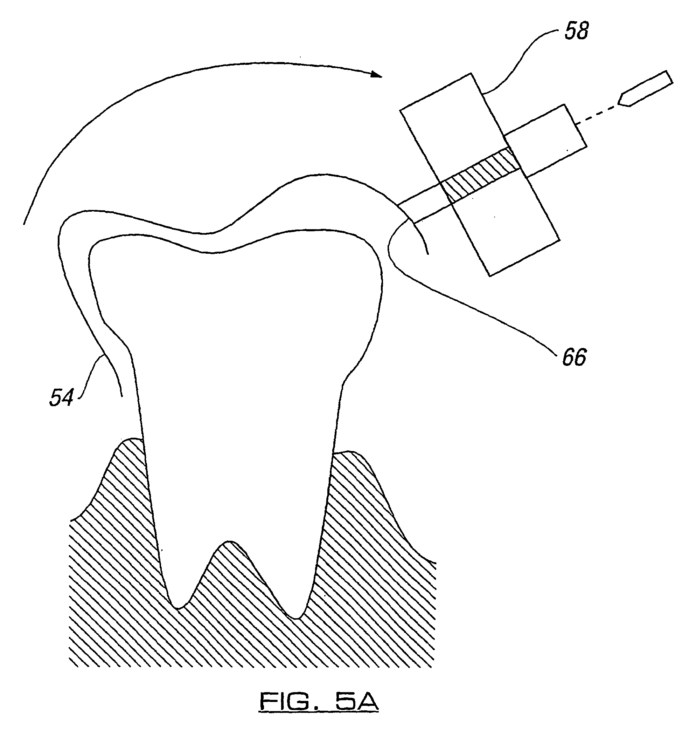

On the buccal side of the frame 50 are provided a plurality of magnets 58, 60, 52, 64 which are retained optionally releasably within collets (not shown) laser welded to a buccal side wall portion 66 disposed on the opposite side of the lattice 52 to the side wall portion 54. The depth of the side wall portion 66 is such that the frame can be rotated into position without lower edge 68 of the side wall portion 66 interfering with the upper surface of any of the teeth over which the frame is disposed (see Figure 5A).

In accordance with a particularly preferred aspect of the invention, the height of the uppermost surfaces of the magnets 58, 60, 62, 64 relative to the lattice 52 which covers the tooth biting surface is carefully selected for both of the pair of frames which in use are fitted to the upper and lower dental quadrants of a wearer on one side of his mouth. Such careful selection ensures that the jaws are constrained to assume an occluded position which is not uncomfortable to the wearer or could induce jaw joint and muscle problems. Ideally, there is substantially planar contact between the contact surfaces of respective adjacent frames applied to maxillary and mandibular dentitions, and the disposition and length of the magnets is such as to maintain the freeway space at the anterior teeth as previously mentioned.

Further in accordance with the invention, one or more interdental screws 68, 70 are screwingly received in small hollow cylinders 72, 74 respectively which are also laser welded to the side wall portion 66. The screws have threads on their outer surfaces which engage corresponding threads on the inner surfaces of the hollow cylinders, and can be tightened so as to displace the sharp conical ends of the screws into the interdental spaces above the tip of the gingival papilla between a pair of the teeth covered by the frame. The tightening of such screws is only effected after the frame has been rotated into place and is firmly seated on the teeth.

In one embodiment, the screws are provided with a hexagonal recess in one end which can receive a suitable sized hexagonal key used for tightening the screws in the cylinders.

Ideally, both magnets and interdental screws and their corresponding cylinders are disposed on the buccal sides of the frames when in place over the teeth.

## Claims

1. A dieting aid comprising at least one pair of frames (14, 16, 26, 28) which are capable of being secured over portions of the maxillary (10, 12) and mandibular dentitions (30, 32) respectively of a wearer, said frames being created using impressions of the maxillary and mandibular jaws so the resulting frames fit snugly over the plurality of teeth for which said frames are made, **characterised in that** inter-dentally extensible and retractable screws (18, 20, 34, 36) are provided in mountings on one side of both frames for releasably securing the frames over said plurality of teeth together with magnetic means (22, 24, 38, 40) also mounted to one side of each of said frames in a disposition such that the magnetic means of the maxillary frame at least partially superpose the magnetic means of the mandibular frame when said frames are secured over the teeth within the mouth of a wearer when the jaws are occluded.

2. A dieting aid according to claim 1 **characterised in that** said frames are created for the dental quadrants of the maxillary and mandibular dentitions.

3. A dieting aid according to either of claim 1 or 2 **characterised in that** the frames are created in such a manner to provide interdentally extending nibs on the lingual side of said frames.

4. A dieting aid according to any preceding claim **characterised in that** the polarities of the magnetic means on the maxillary frame and the mandibular frame are opposite on the surfaces thereof which are more proximate when the frames are in mounted on the teeth within the mouth of the wearer.

5. A dieting aid according to any preceding claim **characterised in that** the magnetic means are split pole magnets.

6. A dieting aid according to any preceding claim **characterised in that** shoulder formations are provided partially or entirely around, or to one side of one of the magnetic means provided on at least one frame secured to one or other of the maxillary or madibular dentition, the magnetic means provided on a second frame secured to the alternate dentition above or below said first magnetic means abutting said shoulder formations when the dentitions are in their substantially occluded condition to prevent significant lateral movement thereof.

7. A dieting aid according to any preceding claim **characterised in that** the frames include buttresses which support the magnetic means.

8. A dieting aid according to any preceding claim **characterised in that** the magnetic means are disposed on the buccal side of the frame as it is situated inside the mouth.

9. A dieting aid according to any preceding claim **characterised in that** the disposition and orientation of the magnetic means on each of the maxillary and mandibular frames is such that when in mating contact ensuring the occlusion of the jaws of the wearer, the so-called freeway space between the biting or occlusal surfaces of at least the anterior teeth is maintained.

10. A dieting aid according to any preceding claim **characterised in that** the frames substantially cover the external surfaces of the teeth of the dental quadrants over which said frames are secured.

11. A dieting aid according to any preceding claim **characterised in that** the magnetic means are laser welded to the frames and are provided with a planar mating surface.

12. A dieting aid according to any of claims 1-10 **characterised in that** the magnetic means are secured to the frame by being set in an acrylic compound mass disposed to one side of the frame.

13. A dieting aid according to claim 12 **characterised in that** the frames are provided with retention lugs on the buccal side of the frame when disposed over the teeth within the mouth by virtue of which the acrylic mass is secured to the frames.

14. A dieting aid according to claim 13 **characterised in that** the acrylic compound mass is tooth coloured.

15. A dieting aid according to claim 13 **characterised in that** the acrylic compound mass is flesh coloured.

16. A dieting aid according to any preceding claim **characterised in that** the frames consist of a tooth biting or occlusal surface cover portion (52), and a side wall portion (54) which depends from or extends upwardly of the cover portion depending on whether the frame is for affixing to a maxillary or mandibular dentition, said side wall portion in use ideally being in contact with the necks of the teeth over which the frame is applied.

17. A dieting aid according to claim 16 **characterised in that** the side wall portion is disposed beneath or above the cover portion on the opposite side of said cover portion to that on which the interdental screws are disposed so that a lateral clamping effect is achieved therewith.

18. A dieting aid according to claim 16 or claim 17 **characterised in that** the cover portion is imperforate.

19. A dieting aid according to claim 16, 17, or 18 **characterised in that** the cover portion is of lattice-type construction.

20. A dieting aid according to any preceding claim **characterised in that** the magnetic means are replaceable.

21. A dieting aid according to claim 20 **characterised in that** said magnetic means consist of magnets having a body which terminates in at least one substantially planar contact surface, said body being received in collet formations secured to the buccal side of the frames.

22. A dieting aid according to claim 21 **characterised in that** the body of the magnets are provided with a screw thread which interengages with a corresponding thread on the inside surface of the collet formations on the frame.

23. A dieting aid according to any preceding claim **characterised in that** each of the frames for either or both of the upper and lower dentitions are created as a single piece and resiliently deformable so as to be capable of snap fitting onto the lingual side of the teeth and over the biting surfaces thereof.

24. A dieting aid according to claim 17 **characterised in that** the side wall portion of the frame allows for the undercut of the teeth with whose sides said side wall portions is in contact to take account of the barrel shape of the teeth above the gums.

25. A dieting aid according to any preceding claim **characterised in that** the frames are created in Titanium.

## Patentansprüche

1. Hilfsmittel zur Gewichtsabnahme, umfassend wenigstens ein Paar Rahmen (14,16, 26, 28), die über Teilen des maxillären (10,12) bzw. mandibularen Gebisses (30,32) eines Trägers befestigt werden können, wobei die Rahmen mit Hilfe von Abdrücken des Ober- und Unterkiefers geschaffen werden, sodass die resultierenden Rahmen satt anliegend auf die Mehrzahl von Zähnen passen, für die die genannten Rahmen angefertigt wurden, **dadurch gekennzeichnet, dass** interdental aus- und einziehbare Schrauben (18, 20, 34, 36) in Halterungen auf einer Seite beider Rahmen zum lösbaren Befestigen der Rahmen auf der genannten Mehrzahl von Zähnen zusammen mit magnetischen Mitteln (22, 24, 38, 40) bereitgestellt sind, die ebenfalls auf einer Seite jeder der genannten Rahmen in einer Anordnung angebracht sind, sodass die magnetischen Mittel des Oberkieferrahmens die magnetischen Mittel des Unterkieferrahmens wenigstens teilweise überlagern, wenn die genannten Rahmen bei Okklusion auf den Zähnen im Mund des Trägers befestigt sind.

2. Hilfsmittel zur Gewichtsabnahme nach Anspruch 1, ferner **dadurch gekennzeichnet, dass** die genannten Rahmen für die Zahnquadranten der Ober- und Unterkiefergebisse geschaffen sind.

3. Hilfsmittel zur Gewichtsabnahme nach Anspruch 1 oder 2, ferner **dadurch gekennzeichnet, dass** die Rahmen so hergestellt sind, dass sie interdental verlaufende Nasen auf der lingualen Seite der genannten Rahmen bereitstellen.

4. Hilfsmittel zur Gewichtsabnahme nach einem der vorhergehenden Ansprüche, **dadurch gekennzeichnet, dass** die Polaritäten der magnetischen Mittel am Oberkieferrahmen und am Unterkieferrahmen auf den Flächen davon entgegengesetzt sind, die näher sind, wenn die Rahmen auf den Zähnen im Mund des Trägers angebracht sind.

5. Hilfsmittel zur Gewichtsabnahme nach einem der vorhergehenden Ansprüche, **dadurch gekennzeichnet, dass** die magnetischen Mittel Spaltpolmagnete sind.

6. Hilfsmittel zur Gewichtsabnahme nach einem der vorhergehenden Ansprüche, **dadurch gekennzeichnet, dass** Schultergebilde an wenigstens einem am Oberkiefer- oder am Unterkiefergebiss befestigten Rahmen teilweise oder völlig um eines der magnetischen Mittel herum oder auf einer Seite davon bereitgestellt sind, wobei das magnetische Mittel, das an einem zweiten Rahmen bereitgestellt ist, der an dem anderen Gebiss über oder unter dem genannten ersten magnetischen Mittel befestigt ist, an Schultergebilden anstößt, wenn die Gebisse in ihrem im Wesentlichen okklusalen Zustand sind, um eine bedeutende seitliche Bewegung davon zu verhindern.

7. Hilfsmittel zur Gewichtsabnahme nach einem der vorhergehenden Ansprüche, **dadurch gekennzeichnet, dass** die Rahmen Stützen aufweisen, die die magnetischen Mittel tragen.

8. Hilfsmittel zur Gewichtsabnahme nach einem der vorhergehenden Ansprüche, **dadurch gekennzeichnet, dass** die magnetischen Mittel auf der bukkalen Seite des im Mund befindlichen Rahmens angeordnet sind.

9. Hilfsmittel zur Gewichtsabnahme nach einem der vorhergehenden Ansprüche, **dadurch gekennzeichnet, dass** die Anordnung und Ausrichtung der magnetischen Mittel am Ober- und Unterkieferrahmen so ist, dass, wenn sie in Gegenkontakt sind, der den Kieferschluss des Trägers gewährleistet, der so genannte interokklusalen Abstand zwischen den Beiß- und Kauflächen von wenigstens den Frontzähnen erhalten bleibt.

10. Hilfsmittel zur Gewichtsabnahme nach einem der vorhergehenden Ansprüche, **dadurch gekennzeichnet, dass** die Rahmen im Wesentlichen die Außenflächen der Zähne der Zahnquadranten bedecken, über denen die genannten Rahmen befestigt sind.

11. Hilfsmittel zur Gewichtsabnahme nach einem der vorhergehenden Ansprüche, **dadurch gekennzeichnet, dass** die magnetischen Mittel an den Rahmen laserangeschweißt sind und mit einer planaren Berührungsfläche versehen sind.

12. Hilfsmittel zur Gewichtsabnahme nach einem der Ansprüche 1 bis 10, **dadurch gekennzeichnet, dass** die magnetischen Mittel am Rahmen befestigt werden, indem sie in eine auf einer Seite des Rahmens angeordneten Acrylverbindungsmasse gesetzt werden.

13. Hilfsmittel zur Gewichtsabnahme nach Anspruch 12, **dadurch gekennzeichnet, dass** die Rahmen mit Halteansätzen auf der bukkalen Seite des Rahmens versehen sind, wenn sie über den Zähnen im Mund angeordnet sind, mithilfe derer die Acrylmasse an den Rahmen befestigt ist.

14. Hilfsmittel zur Gewichtsabnahme nach Anspruch 13, **dadurch gekennzeichnet, dass** die Acrylverbindungsmasse zahnfarben ist.

15. Hilfsmittel zur Gewichtsabnahme nach Anspruch 13, **dadurch gekennzeichnet, dass** die Acrylverbindungsmasse fleischfarben ist.

16. Hilfsmittel zur Gewichtsabnahme nach einem der vorhergehenden Ansprüche, **dadurch gekennzeichnet, dass** die Rahmen einen Biss- oder Okklusionsflächenabdeckteil (52) und einen Seitenwandteil (54) umfassen, der vom Abdeckteil abhängt oder sich von ihm aufwärts erstreckt, je nachdem, ob der Rahmen zum Befestigen an einem maxillären oder mandibularen Gebiss ist, wobei der genannte Seitenwandteil im Gebrauch idealerweise mit den Hälsen der Zähne in Kontakt ist, auf denen dem Rahmen angebracht ist.

17. Hilfsmittel zur Gewichtsabnahme nach Anspruch 16, **dadurch gekennzeichnet, dass** der Seitenwandteil unter oder über dem Abdeckteil auf der entgengesetzten Seite des genannten Abdeckteils zu derjenigen angeordnet ist, auf der die interdentalen Schrauben angeordnet sind, sodass damit ein seitlicher Klemmeffekt erzielt wird.

18. Hilfsmittel zur Gewichtsabnahme nach Anspruch 16 oder Anspruch 17, **dadurch gekennzeichnet, dass** der Abdeckteil ohne Öffnungen ist.

19. Hilfsmittel zur Gewichtsabnahme nach Anspruch 16, 17 oder 18, **dadurch gekennzeichnet, dass** der Abdeckteil einen gitterartigen Aufbau hat.

20. Hilfsmittel zur Gewichtsabnahme nach einem der vorhergehenden Ansprüche, **dadurch gekennzeichnet, dass** die magnetischen Mittel austauschbar sind.

21. Hilfsmittel zur Gewichtsabnahme nach Anspruch 20, **dadurch gekennzeichnet, dass** die genannten magnetischen Mittel Magneten mit einem Körper umfassen, der in wenigstens einer im Wesentlichen planaren Kontaktfläche endet, wobei der genannte Körper in Hülsengebilden aufgenommen ist, die auf der bukkalen Seite der Rahmen befestigt sind.

22. Hilfsmittel zur Gewichtsabnahme nach Anspruch 21, **dadurch gekennzeichnet, dass** der Körper der Magnete mit einem Schraubgewinde versehen ist, das mit einem entsprechenden Gewinde an der Innenfläche der Zwingengebilde am Rahmen in Eingriff kommt.

23. Hilfsmittel zur Gewichtsabnahme nach einem der vorhergehenden Ansprüche, **dadurch gekennzeichnet, dass** die Rahmen jeweils für das obere und/oder das untere Gebiss als einteilige Stücke und federnd verformbar herstellt sind, um auf der lingualen Seite der Zähne und über die Bissflächen davon aufgeschnappt werden zu können.

24. Hilfsmittel zur Gewichtsabnahme nach Anspruch 17, **dadurch gekennzeichnet, dass** der Seitenwandteil des Rahmens die Rückneigung der Zähne berücksichtigt, mit deren Seiten die genannten Seitenwandteile in Kontakt sind, um die Fassform der Zähne über dem Zahnfleisch in Betracht zu ziehen.

25. Hilfsmittel zur Gewichtsabnahme nach einem der vorhergehenden Ansprüche, **dadurch gekennzeichnet, dass** die Rahmen aus Titan angefertigt sind.

## Revendications

1. Dispositif d'aide à un régime amaigrissant comprenant au moins une paire d'armatures (14, 16, 26, 28) qui peuvent être fixées respectivement au-dessus de parties des dentitions maxillaires (10, 12) et mandibulaires (30, 32) de sujets porteurs, lesdites armatures étant créées à partir d'empreintes des mâchoires maxillaires et mandibulaires, de sorte que les armatures résultantes s'adaptent étroitement au-dessus de la pluralité de dents pour lesquelles lesdites armatures sont réalisées, **caractérisé en ce que** des vis interdentaires (18, 20, 34, 36) extensibles et rétractables sont prévues dans des montures sur un côté des deux armatures, pour fixer de façon amovible les armatures au-dessus de ladite pluralité de dents, ainsi que des moyens magnétiques (22, 24, 38, 40) également montés d'un côté de chacune desdites armatures en une disposition telle que les moyens magnétiques de l'armature maxillaire se superposent au moins en partie aux moyens magnétiques de l'armature mandibulaire lorsque lesdites armatures sont fixées au-dessus des dents, dans la bouche d'un sujet porteur, quand les mâchoires sont en position d'occlusion.

2. Dispositif d'aide à un régime amaigrissant selon la revendication 1, **caractérisé en ce que** lesdites armatures sont créées pour les quadrants dentaires de dentitions maxillaires et mandibulaires.

3. Dispositif d'aide à un régime amaigrissant selon la revendication 1 ou 2, **caractérisé en ce que** les armatures sont créées de sorte à fournir des tenons qui s'étendent entre les dents, du côté lingual des desdites armatures.

4. Dispositif d'aide à un régime amaigrissant selon l'une quelconque des revendications précédentes, **caractérisé en ce que** les polarités des moyens magnétiques sur l'armature maxillaire et l'armature mandibulaire sont opposées sur leurs surfaces qui sont les plus proches lorsque les armatures sont en place, montées sur les dents à l'intérieur de la bouche du sujet porteur.

5. Dispositif d'aide à un régime amaigrissant selon l'une quelconque des revendications précédentes, **caractérisé en ce que** les moyens magnétiques sont des aimants de type split-pole.

6. Dispositif d'aide à un régime amaigrissant selon l'une quelconque des revendications précédentes, **caractérisé en ce que** des formations en épaulement sont prévues partiellement ou totalement autour, ou sur un côté, de l'un des moyens magnétiques prévus sur au moins une armature fixée soit à la dentition maxillaire, soit à la dentition mandibulaire, le moyen magnétique prévu sur une seconde armature fixée à l'autre dentition au-dessus ou au-dessous dudit premier moyen magnétique venant buter contre lesdites formations en épaulement lorsque les dentitions sont sensiblement en leur état d'occlusion, pour éviter tout mouvement latéral significatif de celles-ci.

7. Dispositif d'aide à un régime amaigrissant selon l'une quelconque des revendications précédentes, **caractérisé en ce que** les armatures incluent des étais qui supportent les moyens magnétiques.

8. Dispositif d'aide à un régime amaigrissant selon l'une quelconque des revendications précédentes, **caractérisé en ce que** les moyens magnétiques sont disposés sur le côté buccal de l'armature lorsqu'elle est en place à l'intérieur de la bouche.

9. Dispositif d'aide à un régime amaigrissant selon l'une quelconque des revendications précédentes, **caractérisé en ce que** la disposition et l'orientation des moyens magnétiques sur chacune des armatures maxillaires et mandibulaires sont telles que, lorsque en un contact ajusté garantissant l'occlusion des mâchoires du sujet porteur, l'espace "libre" compris entre les surfaces de morsure ou occlusales est conservé, au minimum, entre les dents antérieures.

10. Dispositif d'aide à un régime amaigrissant selon l'une quelconque des revendications précédentes, **caractérisé en ce que** les armatures recouvrent sensiblement les surfaces externes des dents des quadrants dentaires au-dessus desquels lesdites armatures sont fixées.

11. Dispositif d'aide à un régime amaigrissant selon l'une quelconque des revendications précédentes, **caractérisé en ce que** les moyens magnétiques sont soudés au laser sur les armatures et sont pourvus d'une surface de contact plane.

12. Dispositif d'aide à un régime amaigrissant selon l'une quelconque des revendications 1 à 10, **caractérisé en ce que** les moyens magnétiques sont fixés à l'armature par encastrement dans une masse de composé acrylique disposée sur un côté de l'armature.

13. Dispositif d'aide à un régime amaigrissant selon la revendication 12, **caractérisé en ce que** les armatures sont pourvues de griffes de retenue sur le côté buccal de l'armature lorsque celle-ci est en place au-dessus des dents, à l'intérieur de la bouche, griffes au moyen desquelles la masse acrylique est fixée aux armatures.

14. Dispositif d'aide à un régime amaigrissant selon la revendication 13, **caractérisé en ce que** la masse de composé acrylique est de la couleur des dents.

15. Dispositif d'aide à un régime amaigrissant selon la revendication 13, **caractérisé en ce que** la masse de composé acrylique est de couleur chair.

16. Dispositif d'aide à un régime amaigrissant selon l'une quelconque des revendications précédentes, **caractérisé en ce que** les armatures se composent d'une partie de couverture (52) de la surface de morsure ou occlusale des dents, et d'une partie formant paroi latérale (54) qui est dirigée vers le bas ou qui s'étend vers le haut de la partie de couverture selon que l'armature est destinée à être fixée sur une dentition maxillaire ou mandibulaire, ladite partie formant paroi latérale étant idéalement, pendant l'emploi, en contact avec les collets des dents au-dessus desquelles est appliquée l'armature.

17. Dispositif d'aide à un régime amaigrissant selon la revendication 16, **caractérisé en ce que** la partie formant paroi latérale est disposée au-dessous ou au-dessus de la partie de couverture, sur le côté de ladite partie de couverture opposé à celui sur lequel sont disposées les vis interdentaires, de sorte à produire avec celles-ci un effet de serrage latéral.

18. Dispositif d'aide à un régime amaigrissant selon la revendication 16 ou la revendication 17, **caractérisé en ce que** la partie de couverture est **caractérisée par** l'imperforation.

19. Dispositif d'aide à un régime amaigrissant selon la revendication 16, 17 ou 18, **caractérisé en ce que** la partie de couverture est une construction de type en nid d'abeille.

20. Dispositif d'aide à un régime amaigrissant selon l'une quelconque des revendications précédentes, **caractérisé en ce que** les moyens magnétiques sont remplaçables.

21. Dispositif d'aide à un régime amaigrissant selon la revendication 20, **caractérisé en ce que** lesdits moyens magnétiques consistent en des aimants ayant un corps qui se termine en au moins une surface de contact sensiblement plane, ledit corps étant reçu dans des formations en douille fixées au côté buccal des armatures.

22. Dispositif d'aide à un régime amaigrissant selon la revendication 21, **caractérisé en ce que** les corps des aimants sont pourvus d'un filetage qui s'engrène avec un filetage correspondant sur la surface intérieure des formations en douille sur l'armature.

23. Dispositif d'aide à un régime amaigrissant selon l'une quelconque des revendications précédentes, **caractérisé en ce que** chacune des armatures pour la dentition supérieure ou la dentition inférieure, ou les deux, est créée d'une seule pièce et est déformable élastiquement de sorte à pouvoir s'encliquer sur le côté lingual des dents et au-dessus des surfaces de morsure de celles-ci.

24. Dispositif d'aide à un régime amaigrissant selon la revendication 17, **caractérisé en ce que** la partie formant paroi latérale de l'armature est réalisée en fonction de la dépouille des dents avec les côtés desquelles lesdites parties formant paroi latérale sont en contact afin de tenir compte de la forme en baril des dents au-dessus des gencives.

25. Dispositif d'aide à un régime amaigrissant selon l'une quelconque des revendications précédentes, **caractérisé en ce que** les armatures sont créées en titane.
